**Europäisches Patentamt**

**European Patent Office**

**Office européen des brevets**

(19)

(11) Veröffentlichungsnummer: **0 209 154**
**B1**

(12)

# EUROPÄISCHE PATENTSCHRIFT

(45) Veröffentlichungstag der Patentschrift:
27.12.89

(51) Int. Cl.⁴: **G01N 33/577, C12Q 1/40**

(21) Anmeldenummer: 86109899.4

(22) Anmeldetag: 18.07.86

E R R A T U M

(SEITE, SPALTE, ZEILE)
(PAGE, COLUMN, LINE)
(PAGE, COLONNE, LIGNE)

| DIE TEXTSTELLE : | | | LAUTET BERICHTIGT: |
|---|---|---|---|
| TEXT PUBLISHED : | | | SHOULD READ : |
| LE PASSAGE SUIVANT : | | | DEVRAIT ETRE LU : |

| | | | |
|---|---|---|---|
| 1.Process for the specific determination of pancreatic alpha-amylase in body fluids, | 6 | 32 | 1. Process for the specific determination of pancreatic alpha-amylase in the presence of salivary alpha-amylase in body fluids, |

| Tag der Entscheidung über die Berichtigung | ) | | Ausgabe- und Ver- öffentlichungstag: | ) | | Patbl.Nr.) 90/32 |
|---|---|---|---|---|---|---|
| Date of decision on rectification: | ) | 5.6.90 | Issue and publication date: | ) | 8.8.90 | EPB no:) ........ |
| Date de décision portant sur modification: | ) | | Date d'édition et de publication: | ) | | Bull. no:) |

**Europäisches Patentamt**

**European Patent Office**

**Office européen des brevets**

(19)

(11) Veröffentlichungsnummer: **0 209 154**
**B1**

(12)

# EUROPÄISCHE PATENTSCHRIFT

(45) Veröffentlichungstag der Patentschrift:
27.12.89

(21) Anmeldenummer: 86109899.4

(22) Anmeldetag: 18.07.86

(51) Int. Cl.⁴: **G01N 33/577, C12Q 1/40**

(54) Verfahren und Reagenz zur spezifischen Bestimmung von Pankreas-alpha-Amylase.

(30) Priorität: 19.07.85 DE 3525926

(43) Veröffentlichungstag der Anmeldung:
21.01.87 Patentblatt 87/4

(45) Bekanntmachung des Hinweises auf die Patenterteilung:
27.12.89 Patentblatt 89/52

(84) Benannte Vertragsstaaten:
AT BE CH DE FR GB IT LI LU NL SE

(56) Entgegenhaltungen:
EP-A- 0 079 793
EP-A- 0 150 309
DE-A- 3 323 245
DE-A- 3 404 876
DE-A- 3 500 526

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof(DE)

(72) Erfinder: Lenz, Helmut, Dr. rer. nat.,
Waldschmidtstrasse 7, D-8132 Tutzing(DE)
Erfinder: Gerber, Martin, Dr. rer. nat.,
Ignaz-Manz-Strasse 1,
D-8120 Weilheim-Unterhausen(DE)
Erfinder: Albert, Winfried, Dr. phil., Moosstrasse 10,
D-8121 Pähl(DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86(DE)

## Beschreibung

Die Erfindung betrifft ein Verfahren zur spezifischen Bestimmung von Pankreas-alpha-Amylase neben Speichel-alpha-Amylase.

Alpha-Amylase (E.C.3.2.1.1) baut 1,4-d-glucosidisch verknüpfte Oligo- und Polysaccharide überwiegend durch zufällige Hydrolyse der 1,4-alpha-glucosidischen Bindungen zu Maltose und Malto-oligosacchariden ab. Das Enzym hat neben der industriellen Fermentationstechnik erhebliche Bedeutung im Rahmen der klinischen Analytik und Diagnostik. Bei zahlreichen Erkrankungen verändert sich nämlich der alpha-Amylasegehalt in den Körperflüssigkeiten wie Serum, Harn oder Duodenalsekret beträchtlich. Im Körper kommen jedoch im wesentlichen zwei alpha-Amylaseenzyme vor, das Pankreasenzym und das Speichelenzym. Da diagnostische Bedeutung nur dem Pankreasenzym zukommt, stellt sich die Aufgabe, diese beiden (neben selten und nur in geringer Menge auftretenden weiteren Isoenzymen) alpha-Amylasen analytisch zu differenzieren. Die Schwierigkeit besteht hierbei darin, daß die beiden multiplen Formen ähnlichen Aufbau besitzen und immunologisch identisch sind (K. Lorentz, Laboratoriumsblätter 32: 118 (1982)). Zur Eliminierung der Aktivität des Speichelenzyms ist es bekannt, Adsorption an Anionenaustauscher, Hemmung durch ein Weizenprotein oder Elektrophorese bzw. Elektrofokussierung anzuwenden. Diese Verfahren sind jedoch entweder in ihrer Trennwirkung unbefriedigend oder für eine Routinediagnostik zu aufwendig. Unter den genannten Methoden ist allein das in Clin. Chem 28/7, 1525-1527 (l982) beschriebene Verfahren der Hemmung des Enzyms vom Speicheltyp durch einen aus Weizenkeimen gewonnenen Inhibitor mit einem für die Routinediagnose akzeptablen Zeitaufwand verbunden, jedoch ist die Selektivität unbefriedigend. Auch bei der optimalen Inhibitorkonzentration bleiben etwa 13 % der Aktivität des Speicheltyp-Enzyms erhalten, während die Aktivität des Pankreasenzyms auf etwa 81 % verringert wird.

In der EP-A 0 150 309 wird bereits vorgeschlagen, die Pankreas-alpha-Amylase neben der Speichel-alpha-Amylase zu bestimmen, indem man in Gegenwart eines monoklonalen Antikörpers arbeitet, der mit Speichel-alpha-Amylase reagiert und hierbei eine Kreuzreaktivität von 5 % oder weniger gegenüber Pankreas- alpha-Amylase aufweist. Mit diesem monoklonalen Antikörper ist es bei Zusatz eines präzipitierenden Agens auch möglich, einen unlöslichen Komplex mit der Speichel-alpha-Amylase zu bilden, den man aus der Lösung abtrennen kann, so daß nur das Pankreasenzym in der Lösung zurückbleibt und dort bestimmt werden kann. Alternativ ist es möglich, den monoklonalen Antikörper in immobilisierter Form zu verwenden und auf diese Weise die Speichel-Amylase abzutrennen. In beiden Fällen ist es jedoch erforderlich, eine unlösliche Phase zu bilden und von der löslichen Phase zu trennen.

In der DE-A-1 3 500 526.2 wird darüberhinaus ein Verfahren der angegebenen Art offenbart, bei dem anstelle des bindenden monoklonalen Antikörpers der genannten europäischen Patentanmeldung ein spezifisch das Speichel-isoenzym hemmender monoklonaler Antikörper verwendet wird. Hierdurch wird zwar eine weitere Verbesserung der alpha-Amylase-Isoenzymbestimmung erreicht, ohne hierbei eine Phasentrennung vornehmen zu müssen, jedoch betrug die maximal erreichte Hemmung des Speichelenzyms weniger als 97 %, so daß immer noch eine unerwünscht große Ungenauigkeit der Bestimmung anhaftet.

Der Erfindung liegt daher die Aufgabe zugrunde, diesen Nachteil zu beseitigen und ein Verfahren und ein Reagenz zu schaffen, welches eine raschere und einfache, aber noch genauere Bestimmung der Pankreas-alpha-Amylase neben der alpha-Amylase vom Speicheltyp in Körperflüssigkeiten ermöglicht.

Gelöst wird diese Aufgabe erfindungsgemäß durch ein Verfahren zur spezifischen Bestimmung von Pankreas-alpha-Amylase neben Speichel-alpha-Amylase in Körperflüssigkeiten, insbesondere in Serum, Plasma, Duodenalsaft oder Urin, durch Umsetzung mit einem System zum Nachweis von alpha-Amylase in Gegenwart eines monoklonalen Antikörpers, der Speichel-alpha-Amylase inhibiert, welches dadurch gekennzeichnet ist, daß man als Inhibitor einen ersten monoklonalen Antikörper, welcher das Speichelenzym zu mehr als 70%, jedoch weniger als 97% spezifisch hemmt, in Kombination mit einem zweiten monoklonalen Anti-Speichel-alpha-Amylase-Antikörper, der dieses Enzym zu weniger als 10% hemmt, verwendet.

Insbesondere eignen sich für die Erfindung hemmende Antikörper mit <93% Hemmung als erste monoklonale Antikörper und bindende Antikörper mit <5% Hemmung des Speichelenzyms als zweite monoklonale Antikörper. Als unterer Grenzwert der Hemmung sind etwa 70%, vorzugsweise 80% anzusehen, um in der erfindungsgemäßen Kombination eine Hemmung über 97% zu erzielen.

Das erfindungsgemäße Verfahren beruht auf der sehr überraschenden Beobachtung, daß die inhibierende Wirksamkeit eines monoklonalen Antikörpers, welcher das Speichelenzym nicht voll zufriedenstellend hemmt, durch einen lediglich bindenden, aber praktisch nicht oder völlig ungenügend hemmenden monoklonalen Antikörper synergistisch verstärkt wird und eine fast quantitative Hemmung des Speichelenzyms bei 100 % Erhaltung der Aktivität des Pankreasenzyms erreicht werden kann. Außerdem gelingt es, so die Inkubationszeit wesentlich zu verringern.

Für das Verfahren der Erfindung werden als erste monoklonale Antikörper bevorzugt die aus den bei NCACC unter den Nummern (99D12) 84122003 und (89E2) 84122004 hinterlegten (National Collection of Animal Cell Culture, Porton Down, GB) Zellkulturen gewonnenen Antikörper verwendet. Als zweite monoklonale Antikörper werden bevorzugt die in der EP-A 0 150 309 offenbarten bindenden Antikörper der bei NCACC unter den Nummern 84111301, 84111302 hinterlegten Zellkulturen verwendet; besonders bevorzugt wird NCACC 84111301.

Die jeweils nötige Menge an erstem und zweitem Antikörper läßt sich für bestimmte Antikörperpräparate leicht durch wenige Vorversuche feststellen. Vorzugsweise verwendet man mindestens 5 μg/ml, mehr bevorzugt mindestens 20 μg/ml an erstem monoklonalem Antikörper und mindestens 1 μg/ml, mehr bevorzugt mindestens 5 μg/ml an zweitem monoklonalem Antikörper.

Erfindungsgemäß verwendbare monoklonale Antikörper lassen sich erhalten durch Immunisierung von Versuchstieren mit nativer oder modifizierter Speichel-alpha-Amylase, Fusion von B-Lymphocyten der so erhaltenen immunisierten Tiere mit transformierenden Agenzien, Klonierung und Kultivierung der so gebildeten Hybridzellen, welche den monoklonalen Antikörper produzieren und Isolierung des letzteren. Besonders geeignete Tiere für die Herstellung der Speichel-alpha-Amylase-Antikörper sind Ratten und Mäuse. Die Immunisierung erfolgt entweder mit nativer humaner Speichel-alpha-Amylase oder mit modifizierter Speichel-Amylase. Verwendet man natives Enzym, so eignen sich hierzu die handelsüblichen, elektrophoretisch homogenen Präparate. Chemisch modifizierte Speichel-alpha-Amylase läßt sich ebenfalls nach bekannten Methoden der Enzymmodifikation erhalten, wie sie z. B. in der DE-A 25 43 994 näher beschrieben sind. Geeignete Modifizierungsmittel sind beispielsweise N-Bromsuccinimid (NBS) unter Oxidation von Tryptophangruppen am Protein (BBA 143 (1967) 462-472), Carboxymethylierung mit Jodacetat (IAA), die hauptsächlich am Histidin angreift bzw. Nitrierung mit Tetranitromethan (TNM) (J. Biol. Chem. 238 (1963) 3307) sowie Diazotierung mit diazotierter Sulfanilsäure (Meth. Enzymol. 25 (1972) 515-531). Am besten geeignet erwies sich dabei das mit TNM modifizierte Enzym bei Verwendung von Balb/c-Mäusen oder das native Enzym bei Verwendung von AJ-Mäusen.

Die Immunisierung erfolgt durch übliche Verabreichung des nativen oder modifizierten Enzyms, vorzugsweise in Kombination mit Adjuvanz. Bevorzugt wird als Adjuvanz Aluminiumhydroxid zusammen mit Bordatella pertussis. Wird für die Immunisierung native Speichel-alpha-Amylase in AJ-Mäusen oder TNM-modifizierte Speichel-alpha-Amylase in Balb/c-Mäusen verwendet, so erfolgt die Immunisierung vorzugsweise über mindestens 9 Monate mit mindestens 7 Immunisierungen (Injektionen I.P.).

Nach erfolgter Immunisierung werden die B-Lymphocyten der immunisierten Tiere nach üblichen Methoden mit transformierenden Agenzien fusioniert. Beispiele für transformierende Agenzien, die im Rahmen der Erfindung verwendet werden können, sind Myelomazellen, transformierende Viren wie z. B. Epstein-Barr-Virus oder die in der DE-A 3 245 665 beschriebenen Agenzien. Die Fusionierung erfolgt nach dem bekannten Verfahren von Koehler und Milstein (Nature 256 (1975) 495-497). Die hierbei gebildeten Hybridzellen werden in üblicher Weise kloniert, z. B. unter Verwendung eines handelsüblichen Zellsorters, und die erhaltenen Klone, welche den gewünschten monoklonalen Antikörper bilden, gezüchtet. Aufgrund des krebsartigen Wachsens der Hybridzellen lassen sich diese auf unbestimmte Zeit weiterkultivieren und produzieren den gewünschten monoklonalen Antikörper in beliebiger Menge.

Für das erfindungsgemäße Bestimmungsverfahren können die monoklonalen Antikörper als solche oder ihre entsprechende immunologische Eigenschaften aufweisenden Fragmente (Fab-Fragmente) verwendet werden. Unter dem Begriff "monoklonaler Antikörper" werden hier daher auch die Fragmente verstanden. Sowohl der komplette Antikörper, als auch seine Fragmente, können in immobilisierter Form verwendet werden.

Die Bestimmung der alpha-Amylase als solche erfolgt nach den hierfür bekannten Methoden. Da die erfindungsgemäße Kombination monoklonaler Antikörper selektiv die alpha-Amylase vom Speicheltyp hemmt und sie so der Enzymaktivitätsbestimmung entzieht, ohne das Pankreasenzym zu beeinträchtigen, entsprechen die bei der alpha-Amylase-Bestimmung in Gegenwart des monoklonalen Antikörpers erhaltenen Werte der dem Pankreasenzym alleine zukommenden Aktivität.

Bevorzugt wird das erfindungsgemäße Verfahren mit einem System zum Nachweis von alpha-Amylase durchgeführt, welches eine Maltopolyose mit 4 bis 7 Glucoseresten im Molekül, Maltosephosphorylase, β-Phosphoglucomutase, Glucose-6-phosphatdehydrogenase und NAD enthält.

Ein weiteres, im Rahmen der Erfindung bevorzugt verwendetes System zum Nachweis der alpha-Amylase besteht aus Nitrophenylmaltopolyose mit 4 bis 7 Glucoseresten im Molekül und alpha-Glucosidase.

Ein weiteres bevorzugtes Nachweissystem für alpha-Amylase besteht aus mit bestimmbaren Gruppen modifizierter Stärke. Der Begriff modifizierte Stärke umfaßt beispielsweise Stärke, die mit bestimmbaren Gruppen modifiziert ist, z. B. das als "Phadebas" handelsübliche Produkt der Firma Pharmazia, Schweden sowie das in der DE-A 28 12 154 beschriebene Produkt sowie im Abbauverhalten veränderte Stärke, beispielsweise Carboxymethylstärke und Grenzdextrine. Alle diese Systeme sind bekannt und bedürfen daher hier keiner näheren Beschreibung.

Für die Durchführung des erfindungsgemäßen Verfahrens wird die Probeflüssigkeit entweder zuerst mit den erfindungsgemäß verwendeten Antikörpern inkubiert und danach direkt im üblichen Amylasetest eingesetzt oder einer Mischung der Antikörper mit dem Amylasenachweisreagenz ohne Substrat zugegeben und nach der Inkubation durch Substratzusatz gestartet. Die Dauer der Inkubationszeit ist von der Aktivität der eingesetzten Antikörper abhängig und beträgt vorzugsweise 0,5 bis 10, insbesondere etwa 5 Minuten.

Sofern eine Abtrennung der Speichel-alpha-Amylase wünschenswert erscheint, kann einer der monoklonalen Antikörper, sowohl in kompletter Form als auch in Form der Fragmente, auch auf einem festen Träger fixiert vorliegen, beispielsweise auf immunosorptivem Papier oder der Oberfläche von Kunststoffröhrchen bzw. -schläuchen. Auf diese Weise wird die alpha-Amylase vom Speicheltyp an den Träger, also die feste Phase, gebunden.

Den durch die Erfindung erzielten Effekt belegen folgende Versuche:

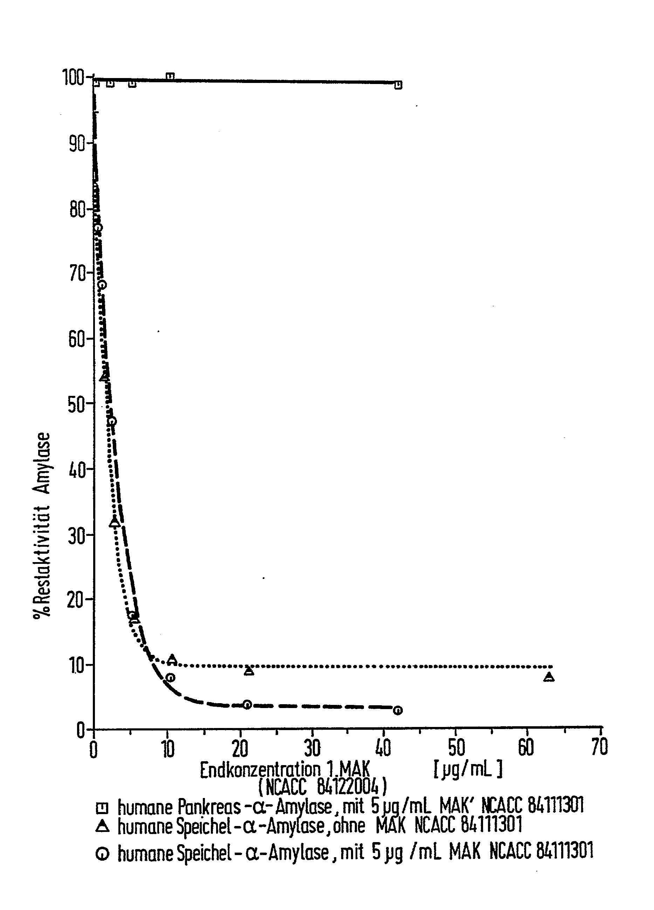

Monoklonale Antikörper (MAK), welche h-SA spezifisch zu maximal 93 % hemmen, werden durch $(NH_4)_2SO_4$-Fällung und DEAE-Chromatographie nach gebräuchlichen Methoden aufgereinigt. Ebenso wird ein MAK, welcher spezifisch h-SA bindet, aufgereinigt. Der oder die monoklonalen Antikörper in Lösung werden mit humaner Amylase vorgemischt und diese Mischung wird 5 Minuten bei Raumtemperatur inkubiert. Danach wird diese Mischung zu Amylasereagenz gegeben und es wird die Amylaseaktivität gemessen. Als Kontrolle dient Amylase, welche mit Puffer ohne MAK's vorgemischt war. Alternativ wird bzw. werden der (die) monoklonale(n) Antikörper in einem Teil des Amylasereagenz - der alpha-Glucosidase-Lösung - vorgelegt. Danach wird die Amylaselösung zugegeben und es wird 5 Minuten inkubiert. Die Reaktion wird mit dem Substrat - $G_7PNP$ (p-Nitrophenylmaltoheptaosid) - gestartet. Als Kontrolle dient eine Glucosidaselösung ohne MAK's. Fig. 1 zeigt die %-Restaktivität der h-SA bzw. h-PA mit und ohne bindenden MAK als Funktion der Endkonzentration an hemmendem MAK, ermittelt mit der Substratstartmethode. In Tabelle 1 sind die Ergebnisse der Serumstartmethode (MAK(s) und Amylase vorgemischt) dargestellt.

Tabelle 1

Hemmung von humaner Speichel- und Pankreas-alpha-Amylase durch hemmenden MAK NCACC 84122003 (MAK I) oder 84122004 (MAK II) und/oder bindendem MAK NCACC 84111301 (MAK III); Serumstartmethode, $G_7PNP$ als Substrat. GA= Gesamtaktivität, A= Aktivität mit MAK(s), RA= % Restaktivität

| Amylase | GA | MAK I 30 µg/ml | | MAK II 25 µg/ml | | MAK III 5 µg/ml | | MAKs I/III 30/5 µg/ml | | MAKs II/III 25/5 µg/ml | |
|---|---|---|---|---|---|---|---|---|---|---|---|
| | | A | RA | A | RA | A | RA | A | RA | A | RA |
| | (U/l) | (U/l) | (%) | (U/l) | (%) | (U/l) | (%) | (U/l) | (%) | (U/l) | (%) |
| Speichel | 1476 | 127 | 8,6 | 135 | 9,1 | 1430 | 96,9 | 40 | 2,7 | 37 | 2,5 |
| Pankreas | 1430 | 1442 | 100,8 | 1428 | 99,9 | 1424 | 99,6 | 1405 | 98,2 | 1426 | 99,7 |

Es zeigt sich anhand der Abbildung und der Tabelle, daß die Kombination von hemmendem, spezifischem MAK sowie spezifisch h-SA bindendem MAK eine synergistische Steigerung der Hemmung bewirkt. Dieser Effekt ist unabhängig vom Substrat. Er wird ebenso bei hochmolekularem Substrat (gefärbte Stärke) als auch bei kurzkettigen Substraten, z.B. $G_5PNP$ (p-Nitrophenylmaltopentaosid) gefunden. Auch beim Substrat eth-$G_7PNP$ wurde der Effekt beobachtet. Auch Speichelamylase in Humanseren wird synergistisch durch die zwei MAK's gehemmt.

Ein weiterer Gegenstand der Erfindung ist ein Reagenz zur spezifischen Bestimmung von Pankreas-alpha-Amylase, neben Speichel-alpha-Amylase in Körperflüssigkeiten, insbesondere in Serum, Duodenalsaft, Plasma oder Urin, enthaltend ein System zum Nachweis von alpha-Amylase und einen monoklonalen Antikörper gegen Speichel-alpha-Amylase, welches dadurch gekennzeichnet ist, daß es einen ersten monoklonalen Antikörper enthält, welcher das Speichelenzym zu mehr als 70%, jedoch weniger als 97% spezifisch hemmt, in Kombination mit einem zweiten monoklonalen Anti-Speichel-alpha-Amylase-Antikörper, der dieses Enzym zu weniger als 10% hemmt, verwendet.

Hinsichtlich des im erfindungsgemäßen Reagenz enthaltenen Systems zum Nachweis von alpha-Amylase und der übrigen Bedingungen gelten die obigen Ausführungen bezüglich des Verfahrens entsprechend.

Die Erfindung ermöglicht eine einfache und besonders rasche und sehr genaue Bestimmung der Pankreas-alpha-Amylase (h-PA) neben alpha-Amylase vom Speicheltyp (h-SA) in Körperflüssigkeiten und verbessert damit die Möglichkeiten der klinischen Diagnostik erheblich.

Die folgenden Beispiele erläutern die Erfindung weiter.

Beispiel 1

Hemmung der humanen Pankreas- und Speichel-alpha-Amylase durch 1. MAK (NCACC 84122003) alleine und in Kombination mit 2. MAK (NCACC 84111301), Serumstartmethode

Reagenzien: Puffer: 100 mM $PO_4^3$, 150 mM NaCl, 6% Rinderserumalbumin, pH 7,1

Amylasereagenz: $G_7PNP$ (Boehringer Mannheim, Kat. Best. Nr. 568 589, 37°C)

MAK–1: 0,63 mg/ml 1. MAK in Puffer

MAK–2: 0,63 mg/ml 1. MAK und 0,105 mg/ml 2. MAK in Puffer

MAK–3: 0,105 mg/ml 2. MAK in Puffer

h–SA: ca. 3000 U/l ($G_7PNP$) in Puffer

h–PA: ca. 3000 U/l (G₇PNP) in Puffer

Versuch: 100 µl h–SA oder h–PA werden mit jeweils 100 µl Puffer oder den verschiedenen MAK-Lösungen gemischt und 5 Minuten bei Raumtemperatur inkubiert. Danach werden jeweils 95 µl dieser Mischungen zu 1000 µl bei 37°C vortemperiertem Amylasereagenz gegeben und die Amylaseaktivität nach Anleitung bestimmt. Die Restaktivität errechnet sich nach folgender Formel:

$$\text{Restaktivität (\%)} = \frac{\text{Akt. mit MAK(s)}}{\text{Akt. ohne MAK(s)}} \times 100$$

Ergebnis: Die entsprechenden Restaktivitäten von h-SA betragen mit 1. MAK 8,6%, mit 2. MAK 96,9% und mit beiden MAK's 2,7%. Die h-PA Restaktivitäten betragen entsprechend 100,8%, 99,6% und 98,2%.

Beispiel 2

Hemmung der Amylase durch 1. MAK (NCACC 84122004) und/oder 2. MAK (NCACC 84111301), Serumstartversion

Reagenzien: wie bei Beispiel 1, nur MAK-1 und MAK-2 verändert.

MAK-1': 0,525 mg/ml 1. MAK in Puffer

MAK-2': 0,525 mg/ml 1. MAK und 0,105 mg/ml 2. MAK in Puffer

Versuch: wie in Beispiel 1, ebenso wie die Berechnung der %-Restaktivität

Ergebnis: Die Restaktivitäten von h-SA betragen mit 1. MAK 9,1 %, mit 2. MAK 96,9 % und mit beiden MAK's 2,5 %. Die h-PA Restaktivitäten betragen entsprechend 99,9 %, 99,6 % und 99,7 %.

Beispiel 3

Hemmung der humanen Speichel- und Pankreas-alpha-Amylase durch 1. MAK (NCACC 84122004) mit und ohne 2. MAK (NCACC 84111301), Substratstartmethode

Reagenzien: Puffer (wie in Beispiel 1)

h-SA (wie in Beispiel 1)

h-PA (wie in Beispiel 1)

MAK-1": 1. MAK in Puffer, verschiedene Konzentrationen

MAK-2": 2. MAK in Puffer, 0.513 mg/ml alpha-Glucosidase: aus Amylasereagenz (Boehringer Mannheim, Kat. Best. Nr. 568589)

Substrat: G₇PNP aus Amylasereagenz

Versuch: Zu 880 µl alpha-Glucosidase werden 10 µl einer MAK-1" Lösung sowie 10 µl MAK-2" Lösung oder 10 µl Puffer zugegeben. Dazu werden 25 µl h-PA oder h-SA gemischt. Diese Mischung wird 5 Minuten bei 37 °C inkubiert. Danach erfolgt der Start durch Zugabe von 100 µl Substratlösung und die Bestimmung der Amylaseaktivität nach Anleitung. Als Kontrolle dient eine Mischung von Alpha-Glucosidase mit 20 µl Puffer sowie der entsprechenden Amylase. Die Aktivität mit MAK(s) dividiert durch die Aktivität ohne MAK's ergibt die %-Restaktivität.

Ergebnis: Fig. 1 zeigt die %-Restaktivität als Funktion der Endkonzentration des 1. MAK bei h-SA mit und ohne 2. MAK und bei h-PA nur mit 2. MAK.

**Patentansprüche**

1. Verfahren zur spezifischen Bestimmung von Pankreas-alpha-Amylase neben Speichel-alpha-Amylase in Körperflüssigkeiten, insbesondere im Serum, Plasma, Duodenalsaft oder Urin, durch Umsetzung mit einem System zum Nachweis von alpha-Amylase in Gegenwart eines monoklonalen Antikörpers, der Speichel-alpha-Amylase inhibiert, dadurch gekennzeichnet, daß man als Inhibitor einen ersten monoklonalen Antikörper, welcher das Speichelenzym zu mehr als 70%, jedoch weniger als 97% spezifisch hemmt, in Kombination mit einem zweiten monoklonalen Anti-Speichel-alpha-Amylase-Antikörper, der dieses Enzym zu weniger als 10% hemmt, verwendet.

2. Verfahren nach Anspruch 1, **dadurch gekennzeichnet,** daß der erste Antikörper das Speichelenzym zu weniger als 93 % und der 2. Antikörper zu weniger als 5 % hemmt.

3. Verfahren nach Anspruch 1 oder 2, **dadurch gekennzeichnet,** daß man monoklonale Antikörper verwendet, die durch Immunisierung von AJ-Mäusen mit nativer oder modifizierter Speichel-alpha-Amylase, Fusion von B-Lymphocyten der immunisierten Tiere mit transformierenden Agentien, Klonierung und Kultivierung der so gebildeten Hybridzellen und Isolierung der monoklonalen Antikörper aus letzteren erhalten werden.

4. Verfahren nach Anspruch 3, **dadurch gekennzeichnet,** daß man als ersten monoklonalen Antikörper NCACC Nr. 84122003 oder NCACC 84122004 verwendet.

5. Verfahren nach Anspruch 2 oder 3, **dadurch gekennzeichnet,** daß man als zweiten monoklonalen Antikörper NCACC Nr. 84111301 oder 84111302 verwendet.

6. Verfahren nach einem der Ansprüche 2 bis 5, **dadurch gekennzeichnet,** daß man mindestens 5 µg/ml an erstem monoklonalem Antikörper und mindestens 1 µg/ml an zweitem monoklonalem Antikörper einsetzt.

7. Verfahren nach einem der Ansprüche 1 bis 6, **dadurch gekennzeichnet,** daß man mindestens 20 µg/ml an erstem monoklonalem Antikörper und mindestens 5 µg/ml an zweitem monoklonalem Antikörper einsetzt.

8. Verfahren nach einem der vorhergehenden Ansprüche, **dadurch gekennzeichnet,** daß als System zum Nachweis von Pankreas-alpha-Amylase eine Maltopolyose mit 4 bis 7 Glucoseresten, Maltose-phosphorylase, β-Phosphoglucomutase, Glucose-6-phosphatdehydrogenase und NAD verwendet wird.

9. Verfahren nach einem der Ansprüche 1 bis 7, **dadurch gekennzeichnet,** daß als System zum Nachweis von Pankreas-alpha-Amylase Nitrophenylmaltopolyose mit 4 bis 7 Glucoseeinheiten im Molekül zusammen mit alpha-Glucosidase verwendet wird.

10. Verfahren nach einem der Ansprüche 1 bis 7, **dadurch gekennzeichnet,** daß als System zum Nachweis von alpha-Amylase Stärke, die mit bestimmbaren Gruppen modifiziert ist, verwendet wird.

11. Reagenz zur spezifischen Bestimmung von Pankreas-alpha-Amylase neben Speichel-alpha-Amylase in Körperflüssigkeiten, insbesondere in Serum, Duodenalsaft, Plasma oder Urin, enthaltend ein System zum Nachweis von alpha-Amylase und einen monoklonalen Antikörper gegen Speichel-alpha-Amylase, dadurch gekennzeichnet, daß es einen ersten monoklonalen Antikörper enthält, welcher das Speichelenzym zu mehr als 70%, jedoch weniger als 97% spezifisch hemmt, in Kombination mit einem zweiten monoklonalen Anti-Speichel-alpha-Amylase-Antikörper, der dieses Enzym zu weniger als 10% hemmt.

12. Reagenz nach Anspruch 11, **dadurch gekennzeichnet,** daß es als System zum Nachweis von Pankreas-alpha-Amylase eine Maltopolyose mit 4 bis 7 Glucoseresten, Maltosephosphorylase, β-Phosphoglucomutase, Glucose-6-phosphatdehydrogenase und NAD enthält.

13. Reagenz nach Anspruch 11, **dadurch gekennzeichnet,** daß es als System zum Nachweis von Pankreas-alpha-Amylase eine Nitrophenylmaltopolyose mit 4 bis 7 Glucoseeinheiten und alpha-Glucosidase enthält.

14. Reagenz nach Anspruch 11, **dadurch gekennzeichnet,** daß es als System zum Nachweis von Pankreas-alpha-Amylase eine mit bestimmbaren Gruppen modifizierte Stärke enthält.

## Claims

1. Process for the specific determination of pancreatic alpha-amylase in body fluids, especially in serum, plasma, duodenal juice or urine, by reaction with a system for the detection of alpha-amylase in the presence of a monoclonal antibody which inhibits salivary alpha-amylase, characterised in that, as inhibitor, there is used a first monoclonal antibody which specifically inhibits the salivary enzyme by more than 70% but less than 97%, in combination with a second monoclonal anti-salivary alpha-amylase antibody which inhibits this enzyme by less than 10%.

2. Process according to claim 1, characterised in that the first antibody inhibits the salivary enzyme by less than 93% and the second antibody by less than 5%.

3. Process according to claim 1 or 2, characterised in that one uses monoclonal antibodies which are obtained by immunisation of AJ mice with native or modified salivary alpha-amylase, fusion of B-lymphocytes of the immunised animals with transforming agents, cloning and culturing of the so formed hybrid cells and isolation of the monoclonal antibodies from the latter.

4. Process according to claim 3, characterised in that, as first monoclonal antibody, one uses NCACC No. 84122003 or NCACC 84122004.

5. Process according to claim 2 or 3, characterised in that, as second monoclonal antibody, one uses NCACC No. 84111301 or 84111302.

6. Process according to one of claims 2 to 5, characterised in that one uses at least 5 µg./ml. of first monoclonal antibody and at least 1 µg./ml. of second monoclonal antibody.

7. Process according to one of claims 1 to 6, characterised in that one uses at least 20 µg./ml. of first monoclonal antibody and at least 5 µg./ml. of second monoclonal antibody.

8. Process according to one of the preceding claims, characterised in that, as system for the detection of pancreatic alpha-amylase, there is used a maltopolyose with 4 to 7 glucose residues, maltose phosphorylase, ß-phosphoglucomutase, glucose-6-phosphate dehydrogenase and NAD.

9. Process according to one of claims 1 to 7, characterised in that, as system for the detection of pancreatic alpha-amylase, there is used nitrophenyl-maltopolyose with 4 to 7 glucose units in the molecule, together with alpha-glucosidase.

10. Process according to one of claims 1 to 7, characterised in that, as system for the detection of alpha-amylase, there is used starch which is modified with determinable groups.

11. Reagent for the specific determination of pancreatic alpha-amylase in the presence of salivary alpha-amylase in body fluids, especially in serum, duodenal juice, plasma or urine, containing a system for the detection of alpha-amylase and a monoclonal antibody against salivary alpha-amylase, characterised in that it contains a first monoclonal antibody which specifically inhibits the salivary enzyme by more than 70% but less than 97%, in combination with a second monoclonal anti-salivary alpha-amylase antibody which inhibits this enzyme by less than 10%.

12. Reagent according to claim 11, characterised in that, as system for the detection of pancreatic alpha-amylase, it contains a maltopolyose with 4 to 7 glucose residues, maltose phosphorylase, ß-phosphoglucomutase, glucose-6-phosphate dehydrogenase and NAD.

13. Reagent according to claim 11, characterised in that, as system for the detection of pancreatic alpha-amylase, it contains a nitrophenylmaltopolyose with 4 to 7 glucose units and alpha-glucosidase.

14. Reagent according to claim 11, characterised in that, as system for the detection of pancreatic alpha-amylase, it contains starch modified with determinable groups.

## Revendications

1. Procéde de détermination spécifique de l'alpha-amylase pancréatique à côté de l'alpha-amylase salivaire dans les fluides corporels, en particulier dans le sérum, le plasma, le suc duodénal ou l'urine, par réaction avec un système pour la mise en évidence de l'alpha-amylase en présence d'un anticorps monoclonal qui inhibe l'alpha-amylase salivaire, caractérisé en ce qu'on utilise comme inhibiteur un premier anticorps monoclonal qui inhibe spécifiquement l'enzyme salivaire à plus de 70%, mais à moins de 97%, en association avec un deuxième anticorps monoclonal anti-alpha-amylase salivaire, qui inhibe cette enzyme à moins de 10%.

2. Procédé selon la revendication 1, caractérisé en ce que le premier anticorps inhibe l'enzyme salivaire à moins de 93% et le deuxième anticorps à moins de 5%.

3. Procédé selon les revendications 1 ou 2, caractérisé en ce qu'on utilise des anticorps monoclonaux qui sont obtenus par immunisation de souris AJ avec de l'alpha-amylase salivaire naturelle ou modifiée, fusion de lymphocytes B des animaux immunisés avec des agents transformants, clonage et culture des cellules hybrides ainsi formées, et isolement des anticorps monoclonaux à partir de ces dernières.

4. Procédé selon la revendication 3, caractérisé en ce qu'on utilise comme premier anticorps monoclonal NCACC No 84122003 ou NCACC 84122004.

5. Procédé selon les revendications 2 ou 3, caractérisé en ce qu'on utilise comme deuxième anticorps monoclonal NCACC No 84111302.

6. Procédé selon l'une des revendications 2 à 5, caractérisé en ce qu'on utilise au moins 5 µg/ml de premier anticorps monoclonal et au moins 1 µg/ml de deuxième anticorps monoclonal.

7. Procédé selon l'une des revendications 1 à 6, caractérisé en ce qu'on utilise au moins 20 mg/ml de premier anticorps monoclonal et au moins 5 µg/ml de deuxième anticorps monoclonal.

8. Procédé selon l'une des revendications précédentes, caractérisé en ce qu'on utilise comme système pour la mise en évidence de l'alpha-amylase pancréatique d'un maltopolyose ayant 4 à 7 radicaux glucose, de la maltose phosphorylase, de la ß-phosphoglucomutase, de la glucose-6-phosphatedéshydrogénase et du NAD.

9. Procédé selon l'une des revendications 1 à 7, caractérisé en ce qu'on utilise comme système pour la mise en évidence de l'alpha-amylase pancréatique un nitrophénylmaltopolyose ayant 4 à 7 motifs glucose dans sa molécule en même temps que de l'alpha-glucosidase.

10. Procédé selon l'une des revendications 1 à 7, caractérisé en ce qu'on utilise comme système pour la mise en évidence de l'alpha-amylase de l'amidon qui a été modifié avec des groupes déterminables.

11. Réactif pour la détermination spécifique de l'alpha-amylase pancréatique à côté de l'alpha-amylase salivaire dans les fluides corporels, en particulier dans le sérum, le suc duodénal, le plasma ou l'urine, contenant un système pour la mise en évidence de l'alpha-amylase et un anticorps monoclonal contre l'alpha-amylase salivaire, caractérisé en ce qu'il contient un premier anticorps monoclonal qui inhibe l'enzyme salivaire à plus de 70%, mais à moins de 97%, en association avec un deuxième anticorps monoclonal anti-alpha-amylase salivaire qui inhibe cette enzyme à moins de 10%.

12. Réactif selon la revendication 11, caractérisé en ce qu'il contient, comme système pour la mise en évidence de l'alpha-amylase pancréatique, un maltopolyose ayant 4 à 7 radicaux glucose, de la maltose phosphorylase, de la ß-phosphoglucomutase, de la glucose-6-phosphatedéshydrogénase et du NAD.

13. Réactif selon la revendication 11, caractérisé en ce qu'il contient comme système pour la mise en évidence de l'alpha-amylase pancréatique, un nitrophénylmaltopolyose ayant 4 à 7 motifs glucose et de l'alpha-glucosidase.

14. Réactif selon la revendication 11, caractérisé en ce qu'il contient comme système pour la mise en évidence de l'alpha-amylase pancréatique, un amidon modifié par des groupes déterminables.

humane Pankreas -α-Amylase, mit 5 μg/mL MAK' NCACC 84111301
humane Speichel-α-Amylase, ohne MAK NCACC 84111301
humane Speichel-α-Amylase, mit 5 μg /mL MAK NCACC 84111301